# EUROPÄISCHE PATENTSCHRIFT

(11) **EP 0 726 252 B1**
(45) Veröffentlichungstag und Bekanntmachung des Hinweises auf die Patenterteilung: **17.04.2002**
(21) Anmeldenummer: 96101300.0
(22) Anmeldetag: 31.01.1996
(51) Int. Cl.: C07D 207/452, C07D 207/40

(54) **Verfahren zur Herstellung von N-substituierten cyclischen Imiden**
Process for the preparation of N-substituted cyclic imides
Procédé pour la préparation d'imides cycliques N-substitués

(30) Priorität: 13.02.1995 DE 19504623
(43) Veröffentlichungstag der Anmeldung: 14.08.1996
(73) Patentinhaber: BAYER AG, 51368 Leverkusen (DE)
(72) Erfinder: Groth, Torsten, Dr., D-51061 Köln (DE); Piejko, Karl-Erwin, Dr., D-51467 Bergisch Gladbach (DE); Joentgen, Winfried, Dr., D-50769 Köln (DE); Käsbauer, Josef, Dr., D-42929 Wermelskirchen (DE); Alig, Bernd, Dr., D-53639 Königswinter (DE); Strüver, Werner, Dr., D-51375 Leverkusen (DE)

(56) Entgegenhaltungen:
- EP-A- 0 165 574
- EP-A- 0 177 031
- EP-A- 0 461 096
- DE-A- 2 100 800
- GB-A- 1 041 027
- US-A- 3 890 270
- US-A- 4 904 803
- PATENT ABSTRACTS OF JAPAN vol. 17, no. 361 (C-1080), 8.Juli 1993 & JP-A-05 051362 (HITACHI), 2.März 1993,

## Beschreibung

Die vorliegende Erfindung betrifft ein besonders vorteilhaftes Verfahren zur Herstellung von N-substituierten cyclischen Imiden, die für die Herstellung von wärmeformbeständigen Kunststoffen und als Zwischenprodukte für Pharmazeutika und Pflanzenschutzmittel verwendet werden können.

Es ist schon lange bekannt, daß man N-substituierte cyclische Imide herstellen kann, indem man ein cyclisches Säureanhydrid mit einem primären Amin in Gegenwart eines sauren Katalysators umsetzt. Die Aufarbeitung der Reaktionsgemische kann durch Zugabe von Säuren und anschließendem Waschen mit Wasser erfolgen (siehe z.B. EP-A 165 574), was natürlich zu organisch belasteten Abwässern führt, die entsorgt werden müssen.

In der US-A 4 904 803 wird eine Aufarbeitung beschrieben, bei der das Reaktionsgemisch zuerst mit einer wäßrigen Base und anschließend mit einer wäßrigen Säure gewaschen wird. Diese wäßrige Aufarbeitung ist nur möglich, wenn zuvor die Reaktion in Gegenwart von Kupfer oder Kupferverbindungen durchgeführt worden ist. Die Abwässer dieses Verfahrens sind nicht nur organisch, sondern auch mit Kupfer belastet, was große Probleme verursacht. Nach eigenen Untersuchungen tritt bei der Verwendung von wäßrigen Basen eine teilweise Hydrolyse der gebildeten Imide auf, was zu Ausbeuteverlusten und Abwässern mit höheren organischen Anteilen führt.

Gemäß der US-A 5 136 052 wird das Reaktionsgemisch verdünnt, ausfallende Niederschläge abfiltriert und das Filtrat destilliert. Dabei wird zwar wasserfrei gearbeitet, aber es ist erforderlich, aus dem Destillationsrückstand durch Extraktion mit Lösungsmitteln anhaftendes Produkt zu entfernen und in das Verfahren zurückzuführen, was verfahrenstechnisch kompliziert ist. Insbesondere fallen große Mengen Filter- und Destillationsrückstände an und es sind viele kostenintensive, verfahrenstechnische Einzeloperationen durchzuführen, was die Kosten sehr ungünstig beeinflußt. Auch die erzielbaren Ausbeuten sind gering.

Schließlich ist aus der GB-PS 1 041 027 bekannt, daß man bei der Umsetzung von Maleinsäureanhydrid mit o-Chloranilin in Abwesenheit eines sauren Katalysators und Aufarbeitung des Reaktionsgemisches unter Zusatz von Natriumbicarbonat N-(2-Chlorphenyl)-maleinimid in einer Ausbeute von lediglich 33 % d.Th. erhält (siehe dort Beispiel 31).

Es besteht daher immer noch das Bedürfnis nach einem Verfahren zur Herstellung von N-substituierten cyclischen Imiden, bei dem man diese möglichst gleichzeitig ohne Abwässer, auf verfahrenstechnisch einfache Weise, in hohen Ausbeuten, in hohen Reinheiten und kostengünstig erhält. Hohe Reinheiten sind bei einer großen Anzahl von N-substituierten cyclischen Imiden erforderlich, wenn sie zur Herstellung von Copolymeren eingesetzt werden sollen.

Es wurde nun ein Verfahren zur Herstellung von N-substituierten cyclischen Imiden der Formel (I) gefunden 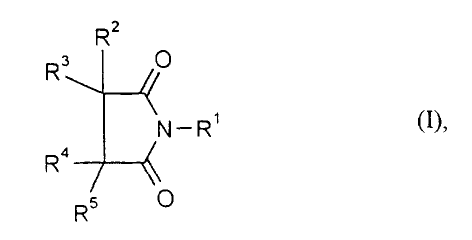 in der
- R¹: für einen C₁-C₂₀-Alkyl-, C₃-C₈-Cycloalkyl-, C₃-C₁₂-Alkenyl-, C₇-C₁₂-Aralkyl- oder C₆-C₁₀-Arylrest, die gegebenenfalls substituiert sein können, oder eine Nitrilgruppe und
- R², R³, R⁴ und R⁵: unabhängig voneinander jeweils für Wasserstoff oder einen C₁-C₁₂-Alkyl- oder C₂-C₁₂-Alkenylrest, die gegebenenfalls substituiert sein können, oder ein Halogen stehen, wobei R² und R³ auch gemeinsam für C₁-C₆-Alkylen stehen können, das gegebenenfalls substituiert sein kann, und R³ und R⁴ gemeinsam auch eine kovalente Bindung bedeuten können
mit der Maßgabe, daß wenigstens eine der drei Bedingungen
a) R² und R³ stehen gemeinsam für C₁-C₆-Alkylen, das gegebenenfalls substituiert sein kann,
b) R³ und R⁴ stehen gemeinsam für eine kovalente Bindung und
c) mindestens einer der Reste R² bis R⁵ steht für C₂-C₁₂-Alkenyl
erfüllt ist,
sowie zur Herstellung von N-substituierten cyclischen Imiden der Formel (Ib) 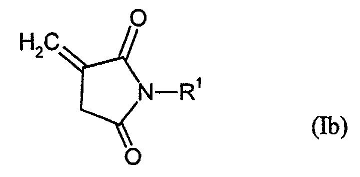 von N-substituierten cyclischen Imiden der Formel (Ic) 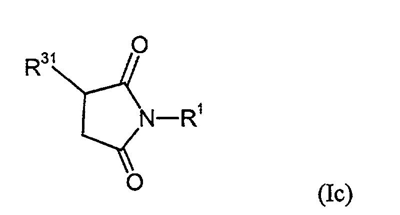 wobei
- R³¹: für Chlor oder C₁-C₄-Alkyl steht,
von N-substituierten cyclischen Imiden der Formel (Id) 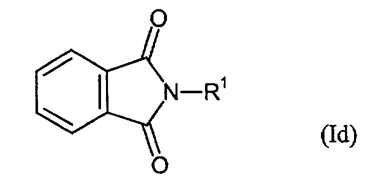 oder deren partiell oder vollständig hydrierten Verbindungen,
wobei
- R¹: in den Formeln (Ib), (Ic) und (Id) jeweils die für die Formel (I) angegebene Bedeutung besitzt,
bei dem man ein cyclisches Säureanhydrid der Formel 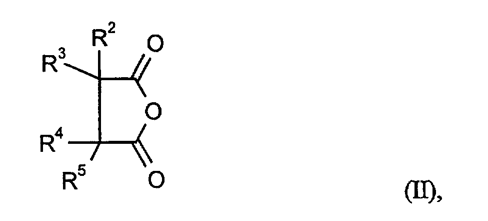 in der
- R² bis R⁵: die bei Formel (I) angegebene Bedeutung haben,
von Itaconsäureanhydrid, Chlorbernsteinsäueanhydrid, C₁-C₄-Alkylbernsteinsäureanhydrid, Phthalsäureanhydrid oder ein partiell oder vollständig hydriertes Phthalsäureanhydrid mit einem Amin der Formel

H₂N-R¹ (III),

in der
- R¹: die bei Formel (I) angegebene Bedeutung hat,
in Gegenwart eines inerten, mit Wasser nicht mischbaren, in Wasser unlöslichen Lösungsmittels, mit dem sich das bei Reaktionstemperatur bildende Wasser aus dem Reaktionsgemisch abtrennen lässt, und eines sauren Katalysators bei 80 bis 200°C und unter Ausschleusung des gebildeten Wassers umsetzt, das dadurch gekennzeichnet ist, daß man ein molares Verhältnis der zuvor genannten Säureanhydride zu Amin (III) von 0,5 - 5:1 einstellt und das Verfahren in Gegenwart eines Stabilisators und eines inerten, dipolaren, aprotischen Cosolvens durchführt, dem nach der Reaktion vorliegenden Reaktionsgemisch gegebenenfalls ein inertes, wenig polares oder unpolares organisches Lösungsmittel zufügt, eine nicht-wäßrige Base, bei der es sich um Ammoniumcarbonat, Ammoniumhydrogencarbonat, Ammoniumcarbonat, Harnstoff oder gasförmigen Ammoniak handelt, in einer Menge von 0,5 bis 50 Gew.-%, bezogen auf das eingesetzte cyclische Anhydrid der zuvor aufgeführten Definitionen, zusetzt, den sich bildenden Niederschlag abtrennt und ein Filtrat erhält, das das N-substituierte cyclische Imid der zuvor genannten Definitionen enthält.

Als Substituenten für alle Alkyl-, Cycloalkyl-, Aralkyl-, Aryl-, Alkenyl- und Alkylenreste kommen beispielsweise in Frage: Halogene wie Fluor, Chlor, Brom und/oder Iod, OH-, NO₂-, NH₂-, CN-, COOH-, C₁-C₄-Alkyl-, C₁-C₄-Alkoxy- und/oder COOC₁-C₆-Alkyl-Gruppen. Von solchen Substituenten können gegebenenfalls z.B. 1 bis 4 Stück pro Molekül der Formeln (I), (II) bzw. (III) vorhanden sein. Als substituierte Alkylgruppe wird Trifluoromethyl besonders erwähnt. Beispiele für C₁-C ₄-Alkylsubstituenten sind iso-Propyl, iso-Butyl und tertiär-Butyl.

Falls weitere NH₂-Gruppen vorliegen, als die im Amin der Formel (III) explizit angegebene, so kann auch an solchen weiteren Aminogruppen eine Cyclisierung mit dem Säureanhydrid der Formel (II) unter Bildung von Bis- oder Polyimiden stattfinden.

Vorzugsweise steht R¹ für unsubstituiertes C₁-C₂₀-Alkyl, C₃-C₆-Cycloalkyl oder Benzyl oder für unsubstituiertes Phenyl oder für Phenyl, das mit 1 bis 3 Substituenten aus der Gruppe C₁-C₄-Alkyl, Fluor, Chlor, Nitro, Hydroxy, Methoxy, Trifluormethyl, Trifluormethoxy und Difluormethoxy substituiert ist.

Soweit R² bis R⁵ Halogen bedeuten ist Fluor, Chlor und Brom, insbesondere Chlor bevorzugt.

Vorzugsweise stehen R² für Wasserstoff, C₁-C₄-Alkyl, C₃-C₄-Alkenyl, Chlor oder Brom, R³ für Wasserstoff, R⁴ für Wasserstoff oder R³ und R⁴ gemeinsam für eine kovalente Bindung und R⁵ für Wasserstoff, C₁-C₄-Alkyl, C₃-C₄-Alkenyl, Chlor oder Brom, wobei entweder mindestens einer der Reste R² und R⁵ für C₃-C₄-Alkenyl oder R ³ und R⁴ gemeinsam für eine kovalente Bindung stehen. Bevorzugt stehen auch R² und R³ gemeinsam für C₁-C₄-Alkylen, R⁴ für Wasserstoff und R⁵ für Wasserstoff, C₁-C₄-Alkyl, C₃-C₄-Alkenyl, Chlor oder Brom.

Besonders bevorzugt einzusetzende Säureanhydride der Formel (II) sind Maleinsäureanhydrid, Monochlormaleinsäureanhydrid, Citraconsäureanhydrid, C₁-C₄-Alkylbernsteinsäureanhydrid und C₂-C₄-Alkenylbernsteinsäureanhydrid. Weitere besonders bevorzugt einzusetzende Säureanhydride sind Phthalsäureanhydrid, Hexahydrophthalsäureanhydrid, 3,4,5,6-Tetrahydrophthalsäureanhydrid, cis-1,2,5,6-Tetrahydrophthalsäureanhydrid, Itaconsäureanhydrid, Chlorbernsteinsäueanhydrid und C₁-C₄-Alkylbernsteinsäureanhydrid.

Besonders bevorzugt einzusetzende Amine der Formel (III) sind Anilin, Methylamin, Ethylamin, tertiär-Butylamin, Hexylamin, Cyclohexylamin, Cyclopropylamin, Laurylamin, Stearylamin, Benzylamin, tertiär-Butylanilin, Chloranilin, Dichloranilin, Nitroanilin, Aminophenol, Anisidin, Methylanilin, Dimethylanilin, Ethylmethylanilin, Trimethylanilin, Fluoranilin, Difluoranilin, Dichlortrifluormethylanilin, Trifluormethylanilin, Trifluormethoxyanilin, Difluormethoxyanilin, Anilinderivate, die mit C₁-C₄-Alkyl, C₁-C₄-Alkoxy, Fluor, Chlor und/oder Brom in 2-, 4- und 5-Stellung substituiert sind und Phenylendiamin. Insbesonders einzusetzende Amine der Formel (III) sind Anilin, 2-Chloranilin, 2,3- 2,4-, 2,5- und 2,6-Dimethylanilin und 2-Ethyl-6-methylanilin.

Das molare Verhältnis von der zuvor definierten Säureanhydride zu Amin der Formel (III) kann z.B. 0,5:1 bis 5:1 betragen. Vorzugsweise beträgt es 0,8:1 bis 1,2:1. Besonders bevorzugt arbeitet man mit äquimolaren Mengen oder einem geringen Überschuß an Säureanhydrid, z.B. bis zu 1,1 Mol Säureanhydrid pro Mol Amin.

Besonders bevorzugt werden folgende N-substituierte cyclische Imide hergestellt: N-Methylmaleinsäureimid, N-Ethylmaleinsäureimid, N-tertiär-Butylmaleinsäureimid, N-Hexylmaleinsäureimid, N-Cyclohexylmaleinsäureimid, N-Cyclopropylmaleinsäureimid, N-Laurylmaleinsäureimid, N-Stearylmaleinsäureimid, N-Benzylmaleinsäureimid, N-Phenylmaleinsäureimid, N-tertiär-Butylphenylmaleinsäureimid, N-Chlorphenylmaleinsäureimid, N-Dichlorphenylmaleinsäureimid, N-Nitrophenylmaleinsäureimid, N-Hydroxyphenylmaleinsäureimid, N-Methoxyphenylmaleinsäureimid, N,N-(Phenylen)-bismaleinsäureimid, N-Methylphenylmaleinsäureimid, N-Dimethylphenylmaleinsäureimid, N-Ethyl-methylphenylmaleinsäureimid, N-Trimethylphenylmaleinsäureimid, N-Fluorphenylmaleinsäureimid, N-Difluorphenylmaleinsäureimid, N-Dichlortrifluormethylphenylmaleinsäureimid, N-Trifluormethylphenylmaleinsäureimid, N-Trifluormethoxymaleinsäureimid, N-Difluormethoxyphenylmaleinsäureimid und die Imide, die den zuvor genannten 2,4,5-trisubstituierten Anilinen entsprechen und die entsprechenden N-substituierten cyclischen Imide, die sich von Monochlormaleinsäureanhydrid, Itaconsäureanhydrid, Citraconsäureanhydrid, Chlorbernsteinsäureanhydrid, C₁-C₄-Alkylbernsteinsäureanhydrid und C₂-C₄-Alkenylbernsteinsäureanhydrid ableiten. Ganz besonders bevorzugt werden N-Phenylmaleinimid, N-2-Chlorphenyl-maleinimid, 2,3-, 2,4-, 2,5- und 2,6-Dimethylphenyl-maleinimid und N-(2-Ethyl-6-methylphenyl)-maleinimid hergestellt.

Als Lösungsmittel werden inerte, mit Wasser nicht mischbare, in Wasser unlösliche organische Lösungsmittel eingesetzt, mit denen sich bei Reaktionstemperatur das sich bildende Wasser aus dem Reaktionsgemisch austragen läßt. Diese Lösungsmittel sind wenig polar oder unpolar. Geeignet sind z.B. Benzol, Toluol, Xylole, Cumol, Mesitylen, Ethylbenzol, Butylbenzol, i-Propylmethylbenzol, t-Butylbenzol, Tetralin, Dekalin, Chlorbenzol, Dichlorbenzole, Anisol und Tetrachlorethan sowie beliebige Mischungen dieser Lösungsmittel.

Als inertes, dipolares, aprotisches Cosolvens kommen z.B. in Frage: N-Methylpyrrolidon, Dioxan, Formamid, N-Methylformamid, Dimethylformamid, Dimethylacetamid, Tetramethylharnstoff, N-Methylcaprolactam, Butyrolacton, Dimethylsulfoxid, Tetramethylensulfon, Hexamethylphosphorsäuretriamid und Ethylenglykoldimethyl- und -diethylether. Bezogen auf das wenig oder unpolare Lösungsmittel kann man z.B. 0,1 bis 20 Gew.-% Cosolvens einsetzen. Vorzugsweise liegt diese Menge bei 0,5 bis 10 Gew.-%, besonders bevorzugt bei 1,5 bis 6 Gew.-%.

Als saure Katalysatoren kommen z.B. die verschiedensten organischen und anorganischen Protonensäuren sowie saure Ionenaustauscher in Frage. Beispiele sind insbesonders Schwefelsäure, Phosphorsäure, Polyphosphorsäuren, Trifluoressigsäure, Trichloressigsäure, Alkylsulfonsäuren wie Methansulfonsäure, Arylsulfonsäuren wie Benzolsulfonsäure, p-Toluolsulfonsäure und Naphthalinsulfonsäure und Ionenaustauscher in der H-Form, die z.B. stark sauer oder schwach sauer und gelförmig oder makroporös sein können, z.B. saure Ionenaustauscher, die von der Bayer AG unter den Bezeichnungen K 1481, K 2441, K 2641, K 2634, VP OC 1052 und VP OC 1501 erhältlich sind. Phosphorsäuren und Ionenaustauscher haben den Vorteil, daß sie aus dem Reaktionsgemisch auf einfache Weise abgetrennt und erneut eingesetzt werden können.

Die sauren Katalysatoren können in handelsüblicher Qualität eingesetzt werden, z.B. Phosphorsäure als 85 %ige Phosphorsäure und wasserhaltige Ionenaustauscher. Wasserhaltige saure Katalysatoren werden zweckmäßigerweise vor der Zugabe des zuvor definierten cyclischen Säureanhydrids azeotrop entwässert.

Saure Katalysatoren können z.B. in Mengen von 0,1 bis 100 Gew.-%, bezogen auf das zuvor definierte eingesetzte Anhydrid, eingesetzt werden. Vorzugsweise liegt diese Menge beim Einsatz von Alkyl- und Arylsulfonsäuren bei 0,1 bis 10 Gew.-%, beim Einsatz von Schwefelsäure bei 0,5 bis 20 Gew.-%, beim Einsatz von Phosphor- und Trihalogenessigsäuren bei 5 bis 40 Gew.-% und beim Einsatz von sauren Ionenaustauschern bei 10 bis 100 Gew.-%, jeweils bezogen auf das zuvor definierte eingesetzte Anhydrid.

Das erfindungsgemäße Verfahren wird in Gegenwart von Stabilisatoren, die auch als Polymerisationsinhibitoren bezeichnet werden, durchgeführt. Beispiele für Polymerisationsinhibitoren sind Phenol und Phenolderivate wie Methoxyphenol, p-tert.-Butylbrenzkatechin, 2,2'-Methylen-bis-(4-methyl-6-tert.-butyl-phenol) und 2,2'-Methylenbis-(4-methyl-6-cyclohexyl-phenol). Polymerisationsinhibitoren können z.B. in Mengen von 0,01 bis 5 Gew.-%, bezogen auf das zuvor definierte eingesetzte Anhydrid, eingesetzt werden. Vorzugsweise liegt diese Menge bei 0,1 bis 2,5 Gew.-%.

Die Reaktionstemperatur liegt vorzugsweise im Bereich 100 bis 180°C, besonders bevorzugt im Bereich 110 bis 160°C. Der Druck ist nicht kritisch. Man kann bei erhöhtem, erniedrigtem oder normalem Druck arbeiten. Vorzugsweise arbeitet man bei Normaldruck und wählt das Lösungsmittel so aus, daß es bei der gewünschten Reaktionstemperatur siedet.

Es ist vorteilhaft, das zuvor definierte Säureanhydrid zusammen mit dem Lösungsmittel, dem Cosolvens, dem Stabilisator und dem sauren Katalysator vorzulegen und das Amin der Formel (III) zuzudosieren. Aus dem Säureanhydrid und dem Amin bildet sich zunächst die entsprechende N-substituierte Amidsäure, die sich im allgemeinen schnell unter Wasserabspaltung in das entsprechende N-substituierte Imid umwandelt. Das gebildete Wasser wird laufend ausgeschleust, z.B. in Form des Azeotrops mit dem jeweils eingesetzten Lösungsmittel. Nach der Abtrennung der Wasserphase aus dem abdestillierten Azeotrop kann die Lösungsmittelphase wieder in die Reaktion zurückgeführt werden.

Außer der oben beschriebenen bevorzugten Reaktionsdurchführung kann man die Umsetzung auch auf andere Weise durchführen, z.B. indem man zunächst weitgehend oder vollständig die N-substituierte Amidsäure bildet und erst dann beginnt Wasser auszuschleusen.

Die Umsetzung ist dann beendet, wenn kein ausschleusbares Wasser mehr anfällt. Es kann vorteilhaft sein, die Ausschleusung von Wasser zu beendigen, wenn noch nicht die theoretisch zu erwartende Menge Wasser ausgeschleust worden ist. Beispielsweise kann man die Ausschleusung von Wasser beenden, wenn mehr als 90 Gew.-%, vorzugsweise mehr als 95 Gew.-% und insbesondere mehr als 97 Gew.-% der theoretisch zu erwartenden Wassermenge ausgeschleust ist.

Wenn das Reaktionsgemisch auf einfache Weise abtrennbare saure Katalysatoren enthält, ist es vorteilhaft, diese vor der eigentlichen Aufarbeitung abzutrennen. Saure Ionenaustauscher kann man z.B. durch Dekantierung oder Filtration abtrennen, Phosphorsäuren liegen im allgemeinen nach dem Abkühlen als abtrennbare Unterphase vor.

Zur Aufarbeitung des Reaktionsgemisches kann man diesem gegebenenfalls zunächst ein inertes, wenig polares oder unpolares organisches Lösungsmittel zufügen. Eine solche Verdünnung ist häufig vorteilhaft, wenn man die Umsetzung in einem relativ konzentrierten Reaktionsgemisch, z.B. in einem Reaktionsgemisch, das nach Beendigung der Reaktion über 35 Gew.-% des gebildeten Imids enthält, durchgeführt hat. Vorzugsweise verwendet man für die Verdünnung das gleiche wenig polare oder unpolare Lösungsmittel wie für die Umsetzung. Die Menge des nach der Umsetzung zuzusetzenden Lösungsmittels kann z.B. 0 bis 200 Gew.-%, bezogen auf das Reaktionsgemisch, betragen. Vorzugsweise beträgt diese Menge 10 bis 150 Gew.-%, insbesondere 30 bis 100 Gew.-%.

Es ist ein wesentliches Merkmal des erfindungsgemäßen Verfahrens, daß man nach der Umsetzung des Säureanhydrids mit dem Amin und gegebenenfalls nach Verdünnung des Reaktionsgemisches mit wenig polarem oder unpolarem Lösungsmittel eine nicht-wäßrige Base mit der zuvor genannten Definition in einer Menge von 0,5 bis 50 Gew.-%, bezogen auf eingesetztes Anhydrid der Formel (II), zusetzt. Vorzugsweise liegt diese Menge bei 1 bis 30 Gew.-%, insbesondere bei 2 bis 20 Gew.-%.

Als nicht-wäßrige Basen werden, wie bereits definiert, Ammoniumcarbonat, Ammoniumhydrogencarbonat, Ammoniumcarbamat, Harnstoff und gasförmiger Ammoniak, letzterer vorzugsweise in verdünnter Form, z.B. im Gemisch mit Stickstoff eingesetzt.

Es ist vorteilhaft, während oder nach der Zugabe der nicht-wäßrigen Base die Temperatur des Reaktionsgemisches zu erniedrigen, beispielsweise auf 10 bis 90°C, vorzugsweise 20 bis 80°C. Besonders bevorzugt gibt man die nicht-wäßrige Base bei 40 bis 80°C zu und trennt den sich bildenden Niederschlag bei 10 bis 50°C ab.

Der sich nach der Zugabe der nicht-wäßrigen Base und der Temperaturerniedrigung bildende Niederschlag wird z.B. durch Filtration abgetrennt. Der abgetrennte Niederschlag kann gegebenenfalls gewaschen werden, z.B. mit einem wenig polaren oder unpolaren Lösungsmittel, und die Waschflüssigkeit dann zusammen mit dem Filtrat aufgearbeitet werden.

Die Gewinnung des hergestellten N-substituierten cyclischen Imids der Formel (I), der Formel (Ib), der Formel (Ic) sowie der Formel (Id) und den partiell oder vollständig hydrierten Verbindungen der Formel (Id) aus dem Filtrat kann auf verschiedene Weise erfolgen. In manchen Fällen erhält man bereits ein ausreichend reines Produkt, wenn man das Filtrat, gegebenenfalls bei vermindertem Druck, teilweise oder bis zur Trockne eindampft. Man kann das Filtrat auch destillativ aufarbeiten, wobei man im allgemeinen zunächst das Lösungsmittel und das Cosolvens erhält, die wiederverwendet werden können, und dann das hergestellte N-substituierte cyclische Imid. Man kann die Destillation gegebenenfalls in Gegenwart eines Polymerisationsinhibitors, beispielsweise eines der oben beschrieben und/oder in Gegenwart von Phosphorsäure, durchführen. Vorzugsweise destilliert man in Gegenwart eines Polymerisationsinhibitors und gegebenenfalls in Gegenwart von Phosphorsäure, besonders bevorzugt in Gegenwart eines Polymerisationsinhibitors und Phosphorsäure.

Man kann auch zunächst aus dem Filtrat das Lösungsmittel abziehen und den Rückstand umkristallisieren. Das bei der Umkristallisation anfallende Filtrat kann gegebenenfalls zurückgeführt werden.

Auch andere Möglichkeiten der Gewinnung des hergestellten N-substituierten cyclischen Imids sind denkbar.

Es ist nicht immer erforderlich, das N-substituierte cyclische Imid der zuvor genannten Definition zu isolieren. Man kann es in manchen Fällen auch in Form des Filtrats, das nach Zugabe der nicht-wäßrigen Base und der Abtrennung des sich dabei bildenden Niederschlags anfällt, weiterverarbeiten.

Gegenüber dem Stand der Technik hat das erfindungsgemäße Verfahren den Vorteil, daß nicht-wäßrig aufgearbeitet wird und deshalb bei der Aufarbeitung, insbesondere der Entsäuerung, keine Abwasserprobleme auftreten. Außerdem werden erfindungsgemäß N-substituierte cyclische Imide in hohen Ausbeuten und Reinheiten erhalten, und Nachbehandlungen von Destillationsrückständen sind nicht erforderlich. Die erfindungsgemäß hergestellten N-substituierten cyclischen Imide weisen einen hohen Qualitätsstandard und einen stark unterdrückten Säuregehalt auf, da saure Katalysatoren, saure Nebenprodukte und restliches Säureanhydrid praktisch vollständig abgetrennt sind. Besonders günstig ist es, daß alle diese Vorteile gleichzeitig erzielt werden. Erfindungsgemäß hergestellte N-substituierte cyclische Imide, insbesondere Maleinimide, sind zur Herstellung von wärmeformbeständigen Kunststoffen besonders geeignet.

### Beispiele

Prozentangaben sind Gewichtsprozente, soweit nichts anderes vermerkt ist.

Folgende Abkürzungen werden verwendet:
NMP = N-Methylpyrrolidon
NPMI = N-Phenylmaleinimid

### Beispiel 1

1 940 g Toluol wurden zusammen mit 120 g NMP, 926 g Maleinsäureanhydrid, 9 g p-Toluolsulfonsäure-Hydrat und 1,8 g p-Methoxyphenol am Wasserabscheider unter Rückfluß gekocht. 838 g Anilin wurden innerhalb von 10 Stunden in das Reaktionsgemisch dosiert und dabei das entstehende Reaktionswasser azeotrop abdestilliert. Nach weiteren 4 Stunden war die Bildung und Abscheidung von insgesamt 180 g Reaktionswasser beendet.

Das Reaktionsgemisch wurde mit 3 864 g Toluol verdünnt, bei 60°C mit 50 g festem Ammoniumcarbonat versetzt und 1,5 Stunden gerührt. Nach Abkühlung auf 30°C wurde der entstandene Niederschlag abfiltriert und mit 460 g Toluol gewaschen. Die Filtrate wurden vereint (7 696 g) und nach Zusatz von 4,5 g 85 %iger Phosphorsäure und 1,5 g eines Polymerisationsinhibitors (Vulkanox® ZKF) im Vakuum destilliert. Folgende Fraktionen wurden erhalten:

| | | |
|---|---|---|
| 1. | 6 066 g | Toluol (wurde im nächsten Ansatz wiederverwendet) |
| 2. | 287 g | NMP/NPMI-Mischfraktion (wurde im nächsten Ansatz wiederverwendet) |
| 3. | 1 178 g | NPMI mit einem Gehalt von 99,4 Gew.-% (HPLC-Analyse). |

Das entsprach einer Ausbeute von 75,1 % der Theorie, bezogen auf eingesetztes Anilin. Die Säurezahl betrug weniger als 0,1 mg KOH/g NPMI, der Schmelzpunkt 89,4°C und der Siedepunkt der Fraktion 3 bei 4 mbar 140 bis 150°C. Eine 47 %ige Lösung dieses NPMIs in Acrylnitril war hellgelb und klar.

### Beispiel 2

Analog Beispiel 1, jedoch mit rückgeführtem Toluol und NMP/NPMI aus Beispiel 1.

2 216 g der Fraktion 1 aus Beispiel 1 wurden zusammen mit den 287 g der Fraktion 2 aus Beispiel 1, 823 g Maleinsäureanhydrid, 9 g p-Toluolsulfonsäure-Hydrat und 1,8 g Methoxyphenol am Wasserabscheider unter Rückfluß gekocht. 745 g Anilin wurden im Verlaufe von 9 Stunden in das Reaktionsgemisch dosiert und dabei das entstandene Reaktionswasser abdestilliert. Nach weiteren 3 Stunden war die Bildung und Abscheidung von insgesamt 157 g Reaktionswasser beendet.

Das Reaktionsgemisch wurde mit 3 583 g Fraktion 1 aus Beispiel 1 verdünnt und sonst wie in Beispiel 1 aufgearbeitet. Die Destillation ergab folgende Fraktionen:

| | | |
|---|---|---|
| 1. | 6 021 g | Toluol (wurde im nächsten Ansatz wiederverwendet) |
| 2. | 307 g | NMP/NPMI-Mischfraktion (wurde im nächsten Ansatz wiederverwendet) |
| 3. | 1 157 g | NPMI mit einem Gehalt von 99,6 Gew.-% (HPLC-Analyse). |

Das entsprach einer Ausbeute von 83,2 % der Theorie. Die Säurezahl betrug 0,1 mg KOH/g NPMI, der Schmelzpunkt und das Aussehen der Lösung in Acrylnitril entsprachen denen des NPMI aus Beispiel 1.

### Beispiel 3

Analog Beispielen 1 und 2, jedoch mit rückgeführtem Toluol und NMP/NPMI aus Beispiel 2.

2 156 g der Fraktion 1 aus Beispiel 2 wurden zusammen mit den 307 g der Fraktion 2 aus Beispiel 2, 823 g Maleinsäureanhydrid, 9 g p-Toluolsulfonsäure-Hydrat und 1,8 g p-Methoxyphenol am Wasserabscheider unter Rückfluß gekocht. Die weitere Arbeitsweise war wie im Beispiel 2 beschrieben, jedoch erfolgte die Anilinzugabe im Verlauf von 10 Stunden, war die Bildung und Abscheidung des Reaktionswassers nach weiteren 2 Stunden beendet, wurden insgesamt 158 g Reaktionswasser ausgeschleust, wurde mit 2 135 g der Fraktion 1 aus Beispiel 2 verdünnt, betrug die Menge der vereinigten Filtrate 6 286 g und ergab die Destillation folgende Fraktionen:

| | | |
|---|---|---|
| 1. | 4 578 g | Toluol (kann wiederverwendet werden) |
| 2. | 286 g | NMP/NPMI-Mischfraktion (kann wiederverwendet werden) |
| 3. | 1 266 g | NPMI mit einem Gehalt von 99,5 Gew.-% (HPLC-Analyse). |

Das entsprach einer Ausbeute von 90,9 % der Theorie. Die Säurezahl betrug 0,5 mg KOH/g NPMI, der Schmelzpunkt 89,2°C und eine 47 %ige Lösung dieses NPMIs in Acrylnitril war hellgelb und klar.

### Beispiel 4 (Vergleichsbeispiel)

### Aufarbeitung ohne Verwendung von Ammoniumcarbonat.

1 673 g Toluol wurden zusammen mit 60 g NMP, 926 g Maleinsäureanhydrid, 9 g p-Toluolsulfonsäure-Hydrat und 1,8 g Methoxyphenol am Wasserabscheider unter Rückfluß gekocht. 838 g Anilin wurden innerhalb von 11 Stunden in das Reaktionsgemisch dosiert und dabei das entstehende Reaktionswasser azeotrop abdestilliert. Nach weiteren 2 Stunden war die Bildung und Abscheidung von insgesamt 180 g Reaktionswasser beendet.

Das Reaktionsgemisch wurde mit 2 215 g Toluol verdünnt, auf 30°C abgekühlt und 1,5 Stunden nachgerührt. Der entstandene Niederschlag wurde abfiltriert und mit 300 g Toluol gewaschen. Die Filtrate wurden vereinigt (5 630 g) und nach Zusatz von 4,5 g 85 %iger Phosphorsäure und 1,5 g Vulkanox® ZKF im Vakuum destilliert. Folgende Fraktionen wurden erhalten.

| | | |
|---|---|---|
| 1. | 3 954 g | Toluol |
| 2. | 237 g | NMP/NPMI-Mischfraktion |
| 3. | 1 228 g | NPMI mit einem Gehalt von 97,1 % (HPLC-Analyse). |

Das entsprach einer Ausbeute von 76,5 % der Theorie. Die Säurezahl betrug 5,0 mg KOH/g NPMI, der Schmelzpunkt 89,2°C und eine 47 %ige Lösung in Acrylnitril war gelb und trübe.

### Beispiel 5

### Mit Dioxan als Cosolvens (statt NMP).

1 900 g Toluol wurden zusammen mit 150 g Dioxan, 926 g Maleinsäureanhydrid, 9 g p-Toluolsulfonsäure-Hydrat und 1,8 g p-Methoxyphenol am Wasserabscheider unter Rückfluß gekocht. 838 g Anilin wurden innerhalb von 13 Stunden in das Reaktionsgemisch dosiert und dabei das entstandene Reaktionswasser azeotrop abdestilliert. Nach weiteren 5 Stunden war die Bildung und Abscheidung von insgesamt 179 g Reaktionswasser beendet.

Das Reaktionsgemisch wurde mit 3 922 g Toluol verdünnt, bei 60°C mit 50 g festem Ammoniumcarbonat versetzt und 1,5 Stunden nachgerührt. Nach Abkühlung auf 30°C wurde der entstandene Niederschlag abfiltriert und mit 400 g Toluol gewaschen. Die Filtrate wurden vereinigt (7 708 g) und nach Zusatz von 4,5 g 85 %iger Phosphorsäure und 1,5 g Vulkanox® ZKF im Vakuum destilliert. Folgende Fraktionen wurden erhalten:

| | | |
|---|---|---|
| 1. | 6 246 g | Toluol/Dioxan-Mischfraktion |
| 2. | 1 360 g | NPMI mit einem Gehalt von 98,8 % (HPLC-Analyse) |

Das entsprach einer Ausbeute von 86,3 % der Theorie. Die Säurezahl betrug weniger als 0,1 mg KOH/g NPMI, der Schmelzpunkt 89,6°C und eine 47 %ige Lösung in Acrylnitril war hellgelb und klar.

### Beispiel 6

### Mit o-Toluidin (statt Anilin).

1 308 g Toluol wurden zusammen mit 80 g NMP, 823 g Maleinsäureanhydrid, 8 g p-Toluolsulfonsäure und 1,8 g p-Methoxyphenol am Wasserabscheider unter Rückfluß gekocht. 857 g o-Toluidin wurden innerhalb von 10 Stunden in das Reaktionsgemisch dosiert und dabei das entstehende Reaktionswasser azeotrop abdestilliert. Nach weiteren 2 Stunden war die Bildung und Abscheidung von insgesamt 160 g Reaktionswasser beendet.

Das Reaktionsgemisch wurde mit 2 550 g Toluol verdünnt, bei 60°C mit 40 g festem Ammoniumcarbonat versetzt und 1,5 Stunden gerührt. Nach Abkühlung auf 30°C wurde der entstandene Niederschlag abfiltriert und mit 300 g Toluol gewaschen. Die Filtrate wurden vereinigt (5 407 g) und nach Zusatz von 4,5 g 85 %iger Phosphorsäure und 1,5 g Vulkanox® ZKF im Vakuum destilliert. Folgende Fraktionen wurden erhalten:

| | | |
|---|---|---|
| 1. | 3 834 g | Toluol |
| 2. | 308 g | NMP/N-2-Methylphenylmaleinimid-Mischfraktion |
| 3. | 1 161 g | N-2-Methylphenylmaleinsäureimid mit einem Gehalt von 99,0 % (HPLC-Analyse). |

Das entspricht einer Ausbeute von 76,6 % der Theorie, bezogen auf eingesetztes o-Toluidin. Die Säurezahl betrug 0,1 mg KOH/g N-(2-Methylphenyl)-maleinsäureimid, der Schmelzpunkt 74,0°C, der Siedepunkt der Fraktion 3 bei 0,6 mbar 142-145°C und eine 47 %ige Lösung in Acrylnitril war hellgelb und klar.

Durch Wiederverwendung der Fraktionen 1 und 2 kann die Ausbeute noch erheblich gesteigert werden (siehe Beispiele 1 bis 3).

### Beispiel 7

### 2-Ethyl-6-methylanilin (statt Anilin).

1 415 g Toluol wurden zusammen mit 90 g NMP, 926 g Maleinsäureanhydrid, 9 g p-Toluolsulfonsäure-Hydrat und 1,8 g p-Methoxyphenol am Wasserabscheider unter Rückfluß gekocht. 1 217 g 2-Ethyl-6-methylanilin wurden innerhalb von 8 Stunden in das Reaktionsgemisch dosiert und dabei das entstehende Reaktionswasser azeotrop abdestilliert. Nach weiteren 1,5 Stunden war die Bildung und Abscheidung von insgesamt 185 g Reaktionswasser beendet.

Das Reaktionsgemisch wurde mit 2 540 g Toluol verdünnt, bei 60°C mit 30 g festem Ammoniumcarbonat versetzt und 1,5 Stunden gerührt. Nach Abkühlung auf 30°C wurde der entstandene Niederschlag abfiltriert und mit 300 g Toluol gewaschen. Die Filtrate wurden vereint (6 251 g) und nach Zusatz von 4,5 g 85 %iger Phosphorsäure und 1,5 g Vulkanox® ZKF im Vakuum destilliert. Folgende Fraktionen wurden erhalten:

| | | |
|---|---|---|
| 1. | 4 228 g | Toluol |
| 2. | 329 g | NMP/N-(2-Ethyl-6-methylphenyl)-maleinimid-Mischfraktion |
| 3. | 1 633 g | N-(2-Ethyl-6-methylphenyl)-maleinsäureimid mit einem Gehalt von 97,8 % (HPLC-Analyse). |

Das entsprach einer Ausbeute von 82,5 % der Theorie, bezogen auf eingesetztes 2-Ethyl-6-methylanilin. Die Säurezahl betrug 0,2 mg KOH/g N-(2-Ethyl-6-methylphenyl)-maleinsäureimid, der Schmelzpunkt 85,9°C, der Siedepunkt der Fraktion 3 bei 1 mbar 148-153°C und eine 47 %ige Lösung in Acrylnitril war hellgelb und klar.

Durch Wiederverwendung der Fraktionen 1 und 2 kann die Ausbeute noch erheblich gesteigert werden (siehe Beispiele 1 bis 3).

### Beispiel 8

### Mit einem stark sauren Ionenaustauscher (statt p-Toluolsulfonsäure)

1 893 g Toluol wurden zusammen mit 151 g NMP, 757 g Maleinsäureanhydrid, 436 g wasserfreiem Ionenaustauscher VP OC 1052 in der H-Form (Bayer AG) und 1,5 g p-Methoxyphenol am Wasserabscheider unter Rückfluß gekocht. 698 g Anilin wurden innerhalb von 6,5 Stunden in das Reaktionsgemisch dosiert und dabei das entstehende Reaktionswasser abdestilliert. Nach weiteren 8,5 Stunden war die Bildung und Abscheidung von insgesamt 146 g Reaktionswasser beendet.

2 403 g des Reaktionsgemisches wurden vom sedimentierten Ionenaustauscher abdekantiert und die verbleibenden 1 338 g des Ionenaustauscher enthaltenden Reaktionsgemisches im nächsten Ansatz wiederverwendet. Das abgetrennte Reaktionsgemisch wurde mit 2 300 g Toluol verdünnt, bei 60°C mit 20 g festem Ammoniumcarbonat versetzt und 1,5 Stunden gerührt. Nach Abkühlung auf 30°C wurde der ausgefallene Niederschlag abfiltriert und mit 170 g Toluol gewaschen. Die Filtrate wurden vereinigt (4 808 g) und im Vakuum destilliert.

Folgende Fraktionen wurden erhalten:

| | | |
|---|---|---|
| 1. | 3 882 g | Toluol (wurde im nächsten Ansatz wiederverwendet) |
| 2. | 117 g | NPMI-haltige Mischfraktion (wurde im nächsten Ansatz wiederverwendet) |
| 3. | 706 g | NPMI mit einem Gehalt von 98,5 % (HPLC-Analyse). |

Das entsprach einer Ausbeute von 53,5 % d. Th. Die Säurezahl betrug 0,2 mg KOH/g NPMI, der Schmelzpunkt 89,4°C, der Siedepunkt der Fraktion 3 bei 4 mbar 137 bis 143°C und eine 47 %ige Lösung in Acrylnitril war hellgelb und klar.

### Beispiel 9

Analog Beispiel 8, jedoch mit rückgeführtem Toluol, NMP/NPMI-Mischfraktion und Ionenaustauscher enthaltendem Reaktionsgemisch aus Beispiel 8.

1 338 g Ionenaustauscher enthaltendes Reaktionsgemisch aus Beispiel 8 wurden zusammen mit 1 552 g Fraktion 1 aus Beispiel 8, 117 g Fraktion 2 aus Beispiel 8, 606 g Maleinsäureanhydrid und 1,2 g Methoxyphenol am Wasserabscheider unter Rückfluß gekocht. 559 g Anilin wurden innerhalb von 7 Stunden in das Reaktionsgemisch dosiert und dabei das entstehende Reaktionswasser abdestilliert. Nach weiteren 8 Stunden war die Bildung und Abscheidung von insgesamt 116 g Reaktionswasser beendet.

2 657 g des Reaktionsgemisches wurden vom sedimentierten Ionenaustauscher dekantiert, mit 2 926 g der Fraktion 1 aus Beispiel 8 verdünnt, bei 60°C mit 20 g festem Ammoniumcarbonat versetzt und 1,5 Stunden gerührt. Nach Abkühlung auf 30°C wurde der entstandene Niederschlag abfiltriert und mit 170 g Toluol gewaschen. Die Filtrate wurden vereint (5 677 g) und im Vakuum unter Zusatz von 1 g Vulkanox® ZKF destilliert. Folgende Fraktionen wurden erhalten:

| | | |
|---|---|---|
| 1. | 4 570 g | Toluol (wurde im nächsten Ansatz wiederverwendet) |
| 2. | 121 g | NPMI-haltige Mischfraktion (wurde im nächsten Ansatz wiederverwendet) |
| 3. | 902 g | N-Phenylmaleinsäureimid mit einem Gehalt von 98,9 % (HPLC-Analyse). |

Das entsprach einer Ausbeute von 85,8 % d. Th. Die Säurezahl betrug 0,2 mg KOH/g NPMI, der Schmelzpunkt 89,5°C, der Siedepunkt der Fraktion 3 bei 4 mbar 137 bis 143°C und eine 47 %ige Lösung in Acrylnitril war hellgelb und klar.

### Beispiele 10 bis 13

Die Beispiele 10 bis 13 wurden analog Beispiel 9 durchgeführt, d.h. unter Verwendung von Toluol, NMP/NPMI-Mischfraktion und Ionenaustauscher enthaltendem Reaktionsgemisch aus dem jeweils vorhergehenden Beispiel.

Die Einsatzmengen von Maleinsäureanhydrid, Methoxyphenol, Anilin, Ammoniumcarbonat und Vulkanox® ZKF entsprachen denen des Beispiels 9. Vor der Vakuumdestillation wurden außerdem noch 3 g 85 %ige Phosphorsäure zu den vereinten Filtraten gegeben.

Es wurden folgende Ergebnisse erhalten:

**Tabelle für Beispiele 10 bis 13**

| | Beispiel Nr. | | | |
|---|---|---|---|---|
| | 10 | 11 | 12 | 13 |
| NPMI (g) | 987 | 974 | 963 | 994 |
| Gehalt nach HPLC-Analyse (%) | 99,5 | 98,5 | 99,1 | 98,9 |
| Ausbeute (% der Theorie) | 94,5 | 92,3 | 91,8 | 94,8 |
| Säurezahl (mg KOH/g NPMI) | 0,2 | 0,2 | 0,2 | 0,2 |
| Schmelzpunkt (°C) | 89,4 | 89,7 | 89,7 | 89,6 |
| | | | | |
| 47 %ige Lösung in Acrylnitril | stets hellgelb und klar | | | |

### Beispiel 14 (zum Vergleich)

### Aufarbeitung ohne Verwendung von Ammoniumcarbonat.

865 g Toluol wurden zusammen mit 64 g NMP, 257 g Maleinsäureanhydrid, 140 g wasserfreiem Ionenaustauscher VP OC 1052 (Bayer AG) in der H-Form und 0,5 g p-Methoxyphenol am Wasserabscheider unter Rückfluß gekocht. 233 g Anilin wurden innerhalb von 4 Stunden in das Reaktionsgemisch dosiert und das entstehende Reaktionswasser abdestilliert. Nach weiteren 2 Stunden war die Bildung und Abscheidung von insgesamt 51 g Reaktionswasser beendet.

1 034 g des Reaktionsgemisches wurden so dekantiert, daß der gesamte Ionenaustauscher im restlichen Reaktionsgemisch verblieb. Das dekantierte Reaktionsgemisch wurde ohne Zugabe von weiterem Toluol und ohne Zugabe von Ammoniumcarbonat im Vakuum destilliert. Es wurden 309 g NPMI mit einem Gehalt von 97,6 % (HPLC-Analyse) erhalten. Das entsprach einer Ausbeute von 69,6 % der Theorie. Die Säurezahl betrug 4,8 mg KOH/g NPMI, der Schmelzpunkt 88,9°C und eine 47 %ige Lösung in Acrylnitril war hellgelb und trübe.

### Beispiel 15

### Mit Phosphorsäure (statt p-Toluolsulfonsäure)

1 800 g Toluol wurden zusammen mit 200 g NMP, 823 g Maleinsäureanhydrid, 170 g wasserfreie Phosphorsäure und 1,6 g p-Methoxyphenol am Wasserabscheider unter Rückfluß gekocht. 745 g Anilin wurden innerhalb von 13 Stunden in das Reaktionsgemisch dosiert und dabei das entstehende Reaktionswasser azeotrop abdestilliert. Nach weiteren 2 Stunden war die Bildung und Abscheidung von insgesamt 149 g Reaktionswasser beendet.

Nach Abkühlung auf 60°C wurden 3 202 g Reaktionsgemisch dekantiert. 341 g Reaktionsgemisch, die den Phosphorsäure-Katalysator enthielten, verblieben im Reaktor und wurden im nächsten Ansatz wieder eingesetzt. Das dekantierte Reaktionsgemisch wurde mit 3 300 g Toluol verdünnt, bei 60°C mit 40 g festem Ammoniumcarbonat versetzt und 2 Stunden gerührt. Nach Abkühlung auf 30°C wurde der entstandene Niederschlag abfiltriert und mit 300 g Toluol gewaschen. Die Filtrate (6 707 g) wurden vereint und nach Zusatz von 4,0 g 85 %iger Phosphorsäure und 1,5 g Vulkanox® ZKF im Vakuum destilliert.

Folgende Fraktionen wurden erhalten:

| | | |
|---|---|---|
| 1. | 5277 g | Toluol (wurde im nächsten Ansatz wiederverwendet) |
| 2. | 418 g | NPMI-haltige Mischfraktion (wurde im nächsten Ansatz wiederverwendet) |
| 3. | 793 g | NPMI mit einem Gehalt von 97,4 % (HPLC-Analyse). |

Das entsprach einer Ausbeute von 55,7 % d. Th. Die Säurezahl betrug 0,3 mg KOH/g NPMI und der Schmelzpunkt 89,5°C.

### Beispiel 16

Analog Beispiel 15, jedoch mit rückgeführtem Toluol, NPMI-haltiger Mischfraktion und katalysatorhaltigem Reaktionsgemisch aus Beispiel 15.

2 067 g der Fraktion 1 aus Beispiel 15 wurden zusammen mit 418 g der Fraktion 2 aus Beispiel 15, 823 g Maleinsäureanhydrid, 341 g des verbliebenen Reaktionsgemisches aus Beispiel 15 und 1,6 g p-Methoxyphenol am Wasserabscheider unter Rückfluß gekocht. 745 g Anilin wurden innerhalb von 11,5 Stunden in das Reaktionsgemisch dosiert und das entstehende Reaktionswasser azeotrop abdestilliert. Nach weiteren 4 Stunden war die Bildung und Ausschleusung von insgesamt 158 g Reaktionswasser beendet.

Nach Abkühlung auf 60°C wurden 3 931 g Reaktionsgemisch dekantiert. 298 g Phosphorsäure enthaltendes Reaktionsgemisch blieben im Reaktor. Das dekantierte Reaktionsgemisch wurde mit 4 000 g eines Gemisches aus der restlichen Fraktion 1 des Beispiels 15 und Toluol verdünnt, bei 60°C mit 40 g festem Ammoniumcarbonat versetzt und 2 Stunden gerührt. Nach Abkühlung auf 30°C wurde der entstandene Niederschlag abfiltriert und mit 300 g Toluol gewaschen. Die Filtrate wurden vereint (8 205 g) und nach Zusatz von 4,0 g 85 %iger Phosphorsäure und 1,5 g Vulkanox® ZKF im Vakuum destilliert.

Folgende Fraktionen wurden erhalten:

| | | |
|---|---|---|
| 1. | 6 393 g | Toluol |
| 2. | 370 g | NPMI-haltige Mischfraktion |
| 3. | 1 169 g | NPMI mit einem Gehalt von 98,6 % (HPLC-Analyse). |

Das entsprach einer Ausbeute von 83,2 % d. Th. Die Säurezahl betrug 0,1 mg KOH/g NPMI, der Schmelzpunkt 89,7°C und eine 47 %ige Lösung in Acrylnitril war hellgelb und klar.

### Beispiel 17

### Mit 2,6-Dichloranilin statt Anilin

384 g Toluol wurden zusammen mit 25 g NMP, 257 g Maleinsäureanhydrid, 2,5 g p-Toluolsulfonsäure-Hydrat und 0,5 g p-Methoxyphenol am Wasserabscheider unter Rückfluß gekocht. 406 g 2,6-Dichloranilin, gelöst in 135 g Toluol, wurden innerhalb von 12 Stunden in das Reaktionsgemisch dosiert und dabei das entstehende Reaktionswasser azeotrop abdestilliert. Nach weiteren 11 Stunden war die Bildung und Abscheidung von insgesamt 53 g Reaktionswasser beendet.

Das Reaktionsgemisch wurde mit 500 g Toluol verdünnt, bei 60°C mit 11 g festem Ammoniumcarbonat versetzt und 2 Stunden gerührt. Nach Abkühlung auf 40°C wurde der entstandene Niederschlag abfiltriert und mit 300 g Toluol nachgewaschen. Die Filtrate wurden vereint (2080 g). Sie enthielten N-(2,6-Dichlorphenyl)-maleinsäureimid in einer Menge, die einer Ausbeute von 92,8 % der Theorie entsprach (HPLC-Analyse; bezogen auf Dichloranilin). Durch fraktioniertes Einengen der Filtrate wurde das N-(2,6-Dichlorphenyl)-maleinsäureimid kristallisiert.

Folgende Fraktionen wurden erhalten:

| Fraktion | 1 | 2 | 3 | Σ Fr. 1-3 |
|---|---|---|---|---|
| Menge (g) | 156,4 | 161,5 | 126,1 | 444,0 |
| Gehalt nach HPLC-Analyse (%) | 99,3 | 97,1 | 94,9 | - |
| Ausbeute (% d.Th. bez. auf 2,6-Dichloranilin) | 25,7 | 25,9 | 19,8 | 71,4 |
| Schmelzpunkt (°C) | 135 | 135 | 134 | - |

Die verbleibende Mutterlauge enthielt nach der dritten Kristallisation noch 17,6 % der Theorie Produkt und das Cosolvens. Zur Vermeidung von Isolierausbeuteverlusten und zur Einsparung von Cosolvens kann die Mutterlauge bei einem Folgeansatz vorgelegt werden.

## Patentansprüche

1. Verfahren zur Herstellung von N-substituierten cyclischen Imiden der Formel 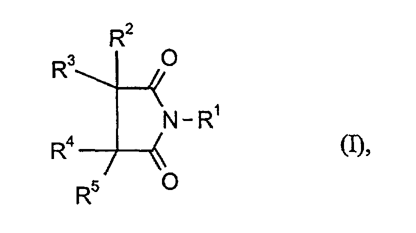 in der
R¹ für einen C₁-C₂₀-Alkyl-, C₃-C₈-Cycloalkyl-, C₃-C₁₂-Alkenyl-, C₇-C₁₂-Aralkyl- oder C₆-C₁₀-Arylrest, die gegebenenfalls substituiert sein können, oder eine Nitrilgruppe und
R², R³, R⁴ und R⁵ unabhängig voneinander jeweils für Wasserstoff oder einen C₁-C₁₂-Alkyl- oder C₂-C₁₂-Alkenylrest, die gegebenenfalls substituiert sein können, oder ein Halogen stehen, wobei R² und R³ auch gemeinsam für C₁-C₆-Alkylen stehen können, das gegebenenfalls substituiert sein kann, und R³ und R⁴ gemeinsam auch eine kovalente Bindung bedeuten können
mit der Maßgabe, daß wenigstens eine der drei Bedingungen
a) R² und R³ stehen gemeinsam für C₁-C₆-Alkylen, das gegebenenfalls substituiert sein kann,
b) R³ und R⁴ stehen gemeinsam für eine kovalente Bindung und
c) mindestens einer der Reste R² bis R⁵ steht für C₂-C₁₂-Alkenyl
erfüllt ist,
von N-substituierten cyclischen Imiden der Formel (Ib) 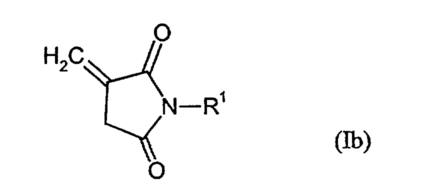 von N-substituierten cyclischen Imiden der Formel (Ic) 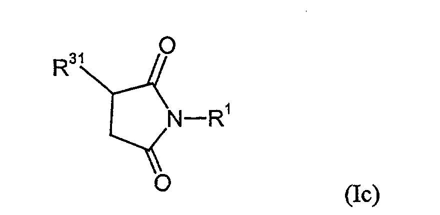 wobei
R³¹ für Chlor oder C₁-C₄-Alkyl steht,
von N-substituierten cyclischen Imiden der Formel (Id) 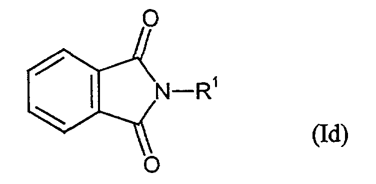 oder deren partiell oder vollständig hydrierten Verbindungen,
wobei
R¹ in den Formeln (Ib), (Ic) und (Id) jeweils die für die Formel (I) angegebene Bedeutung besitzt,
durch Umsetzung eines cyclischen Säureanhydrids der Formel 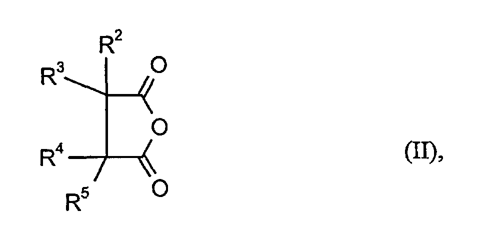 in der
R² bis R⁵ die bei Formel (I) angegebene Bedeutung haben,
von Itaconsäureanhydrid, Chlorbernsteinsäureanhydrid, C₁-C₄-Alkylbernsteinsäureanhydrid, Phthalsäureanhydrid oder einem partiell oder vollständig hydrierten Phthalsäureanhydrid mit einem Amin der Formel
H₂N-R¹ (III),
in der
R¹ die bei Formel (I) angegebene Bedeutung hat,
in Gegenwart eines inerten, mit Wasser nicht mischbaren, in Wasser unlöslichen Lösungsmittels, mit dem sich das bei Reaktionstemperatur bildende Wasser aus dem Reaktionsgemisch abtrennen läßt, und eines sauren Katalysators bei 80 bis 200°C und unter Ausschleusung des gebildeten Wassers, **dadurch gekennzeichnet, daß** man ein molares Verhältnis der zuvor genannten cyclischen Säureanhydride zu Amin (III) von 0,5 - 5:1 einstellt und das Verfahren in Gegenwart eines Stabilisators und eines inerten, dipolaren, aprotischen Cosolvens durchführt, dem nach der Reaktion vorliegenden Reaktionsgemisch gegebenenfalls ein inertes, wenig polares oder unpolares organisches Lösungsmittel zufügt, eine nicht-wäßrige Base, bei der es sich um Ammoniumcarbonat, Ammoniumhydrogencarbonat, Ammoniumcarbamat, Harnstoff oder gasförmigen Ammoniak handelt, in einer Menge von 0,5 bis 50 Gew.-%, bezogen auf das eingesetzte cyclische Anhydrid der zuvor aufgeführten Definitionen zusetzt, den sich bildenden Niederschlag abtrennt und ein Filtrat erhält, das das N-substituierte cyclische Imid der zuvor genannten Definitionen enthält.

2. Verfahren nach Anspruch 1, **dadurch gekennzeichnet, daß** in den Formeln (I), (II) und (III) Alkyl-, Cycloalkyl-, Aralkyl-, Aryl-, Alkenyl- und Alkylenylreste mit Fluor, Chlor, Brom, Iod, OH-, NO₂-, NH₂-, CN-, COOH-, C₁-C₄-Alkyl-, C₁-C₄-Alkoxy- und/oder COOC₁-C₆-Alkyl-Gruppen substituiert sind, bevorzugt **dadurch gekennzeichnet, daß** in den Formeln (I) und (III) R¹ für unsubstituiertes C₁-C₂₀-Alkyl, C₃-C₆-Cycloalkyl oder Benzyl oder für unsubstituiertes Phenyl oder für Phenyl, das mit 1 bis 3 Substituenten aus der Gruppe C₁-C₄-Alkyl, Fluor, Chlor, Nitro, Hydroxy, Methoxy und Trifluormethyl substituiert ist, steht und
daß in den Formeln (I) und (II)
R² für Wasserstoff, C₁-C₄-Alkyl, C₃-C₄-Alkenyl, Chlor oder Brom und
R³ für Wasserstoff,
R⁴ für Wasserstoff oder
R³ und R⁴ gemeinsam für eine kovalente Bindung und
R⁵ für Wasserstoff, C₁-C₄-Alkyl, C₃-C₄-Alkenyl, Chlor oder Brom stehen,
wobei entweder mindestens einer der Reste R² und R⁵ für C₃-C₄-Alkenyl oder R³ und R⁴ gemeinsam für eine kovalente Bindung oder R² und R³ gemeinsam für C₁-C₄-Alkylen, R⁴ für Wasserstoff und R⁵ für Wasserstoff, C₁-C₄-Alkyl, C₃-C₄-Alkenyl, Chlor oder Brom stehen.

3. Verfahren nach Ansprüchen 1 und 2, **dadurch gekennzeichnet, daß** man als Lösungsmittel Benzol, Toluol, Xylole, Cumol, Mesitylen, Ethylbenzol, Butylbenzol, i-Propylmethylbenzol, t-Butylbenzol, Tetralin, Dekalin, Chlorbenzol, Dichlorbenzole oder Anisol sowie beliebige Mischungen dieser Lösungsmittel untereinander unter Zusatz von 0,1 bis 20 Gew.-% eines Cosolvens einsetzt, das aus N-Methylpyrrolidon, Dioxan, Formamid, N-Methylformamid, Dimethylformamid, Dimethylacetamid, Tetramethylharnstoff, N-Methylcaprolacton, Butyrolacton, Dimethylsulfoxid, Tetramethylensulfon, Hexamethylphosphorsäuretriamid und Ethylenglykoldimethyl- und -diethylether ausgewählt ist.

4. Verfahren nach Ansprüchen 1 bis 3, **dadurch gekennzeichnet, daß** man als saure Katalysatoren Schwefelsäure, Phosphorsäure, Polyphosphorsäuren, Trifluoressigsäure, Trichloressigsäure, Alkylsulfonsäuren, Arylsulfonsäuren und/oder Ionenaustauscher in der H-Form in einer Menge von 0,1 bis 100 Gew.-%, bezogen auf das eingesetzte Anhydrid der in Anspruch 1 genannten Definitionen, einsetzt.

5. Verfahren nach Ansprüchen 1 bis 4, **dadurch gekennzeichnet, daß** man als Stabilisatoren Phenol oder Phenolderivate in Mengen von 0,01 bis 5 Gew.-%, bezogen auf das eingesetzte Anhydrid der in Anspruch 1 genannten Definitionen, einsetzt.

6. Verfahren nach Ansprüchen 1 bis 5, **dadurch gekennzeichnet, daß** man die in Anspruch 1 definierte nicht-wäßrige Base in einer Menge von 0,5 bis 50 Gew.-%, bezogen auf eingesetztes Anhydrid der in Anspruch 1 genannten Definitionen, einsetzt.

7. Verfahren nach Ansprüchen 1 bis 6, **dadurch gekennzeichnet, daß** man während oder nach der Zugabe der in Anspruch 1 definierten nicht-wäßrigen Base die Temperatur des Reaktionsgemisches auf 10 bis 90°C erniedrigt.

8. Verfahren nach Ansprüchen 1 bis 7, **dadurch gekennzeichnet, daß** man die hergestellten N-substituierten cyclischen Imide der in Anspruch 1 genannten Definitionen aus dem Filtrat, das nach der Abtrennung des nach Zusatz der in Anspruch 1 definierten nicht-wäßrigen Base gebildeten Niederschlags vorliegt, durch Eindampfen, destillative Aufarbeitung oder Abziehen des Lösungsmittels und Umkristallisation des Rückstands gewinnt.

9. Verfahren nach Ansprüchen 1 bis 8, **dadurch gekennzeichnet, daß** man als saure Katalysatoren Ionenaustauscher in der H-Form oder Phosphorsäuren verwendet, diese aus dem Reaktionsgemisch abtrennt und erneut einsetzt.

10. Verfahren nach Ansprüchen 1 bis 9, **dadurch gekennzeichnet, daß** man das Filtrat, das das N-substituierte cyclische Imid der in Anspruch 1 genannten Definitionen enthält, destillativ aufarbeitet und die Lösungsmittel und Cosolvens enthaltenden Fraktionen wiederverwendet.

## Claims

1. Process for the preparation of N-substituted cyclic imides of the formula 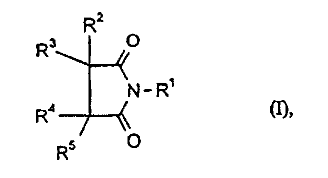 in which
R¹ is a C₁-C₂₀-alkyl, C₃-C₈-cycloalkyl, C₃-C₁₂-alkenyl, C₇-C₁₂-aralkyl or C₆-C₁₀-aryl radical, each of which can optionally be substituted, or a nitrile group and
R², R³, R⁴ and R⁵ independently of one another are each hydrogen, a C₁-C₁₂-alkyl or C₂-C₁₂-alkenyl radical, each of which can optionally be substituted, or a halogen, it also being possible for R² and R³ together to be C₁-C₆-alkylene which can optionally be substituted, and for R³ and R⁴ together to be a covalent bond,
with the proviso that at least one of the following three conditions is satisfied:
a) R² and R³ together are C₁-C₆-alkylene which can optionally be substituted,
b) R³ and R⁴ together are a covalent bond and
c) at least one of the radicals R² to R⁵ is C₂-C₁₂-alkenyl,
of N-substituted cyclic imides of the formula (Ib) 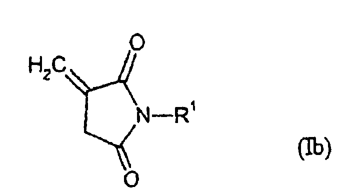 of N-substituted cyclic imides of the formula (Ic) 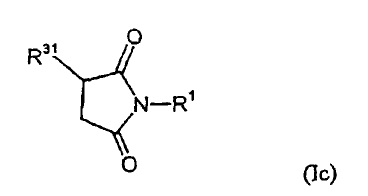 where
R³¹ is chlorine or C₁-C₄-alkyl,
of N-substituted cyclic imides of the formula (Id) 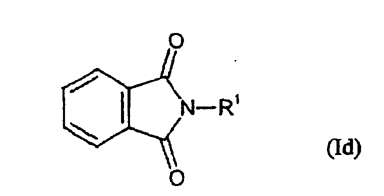 or their partially or completely hydrogenated compounds,
where
R¹ in each of the formulae (Ib), (Ic) and (Id) has the meaning defined for R¹ in the formula I,
by reaction of a cyclic acid anhydride of the formula 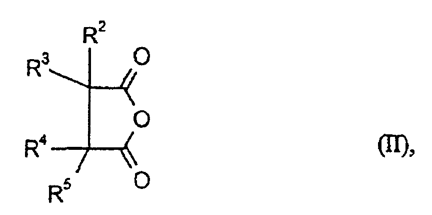 in which
R² to R⁵ are as defined for the formula (I),
of itaconic anhydride, chlorosuccinic anhydride, C₁-C₄-alkylsuccinic anhydride, phthalic anhydride or a partially or completely hydrogenated phthalic anhydride with an amine of the formula
H₂N-R¹ (III),
in which
R¹ is as defined for the formula (I),
in the presence of an inert water-immiscible water-insoluble solvent with which the water formed at reaction temperature can be separated from the reaction mixture and an acid catalyst, at 80 to 200°C and with removal of the water formed, **characterized in that** the molar ratio of the abovementioned cyclic acid anhydride to amine (III) is adjusted to 0.5 - 5:1 and the process is carried out in the presence of a stabilizer and an inert, dipolar, aprotic cosolvent, an inert organic solvent of low or zero polarity is optionally added to the reaction mixture present after the reaction, a non-aqueous base selected from the group consisting of ammonium carbonate, ammonium hydrogencarbonate, ammonium carbamate, urea and gaseous ammonia, is added in an amount of 0.5 to 50% by weight, based on the above-defined cyclic anhydride used, and the precipitate formed is separated off to give a filtrate containing the N-substituted cyclic imide defined above.

2. Process according to Claim 1, **characterized in that**, in the formulae (I), (II) and (III), alkyl, cycloalkyl, aralkyl, aryl, alkenyl and alkylene radicals are substituted by fluorine, chlorine, bromine, iodine or OH, NO₂, NH₂, CN, COOH, C₁-C₄-alkyl, C₁-C₄-alkoxy and/or COOC₁-C₆-alkyl groups, preferably **characterized in that**, in the formulae (I) and (III), R¹ is unsubstituted C₁-C₂₀-alkyl, C₃-C₆-cycloalkyl or benzyl, unsubstituted phenyl or phenyl substituted by 1 to 3 substituents from the group consisting of C₁-C₄- alkyl, fluorine, chlorine, nitro, hydroxyl, methoxy and trifluoromethyl, and
**in that**, in the formulae (I) and (II),
R² is hydrogen, C₁-C₄-alkyl, C₃-C₄-alkenyl, chlorine or bromine,
R³ is hydrogen,
R⁴ is hydrogen, or
R³ and R⁴ together are a covalent bond, and
R⁵ is hydrogen, C₁-C₄-alkyl, C₃-C₄-alkenyl, chlorine or bromine,
either at least one of the radicals R² and R⁵ being C₃-C₄-alkenyl or R³ and R⁴ together being a covalent bond, or R² and R³ together being C₁-C₄-alkylene, R⁴ being hydrogen and R⁵ being hydrogen, C₁-C₄-alkyl, C₃-C₄- alkenyl, chlorine or bromine.

3. Process according to Claims 1 and 2, **characterized in that** the solvents used are benzene, toluene, xylenes, cumene, mesitylene, ethylbenzene, butylbenzene, i-propylmethylbenzene, t-butylbenzene, tetralin, decalin, chlorobenzene, dichlorobenzenes or anisole, and also any desired mixtures of these solvents with one another, with the addition of 0.1 to 20% by weight of a cosolvent selected from N-methylpyrrolidone, dioxane, formamide, N-methylformamide, dimethylformamide, dimethylacetamide, tetramethylurea, N-methylcaprolactone, butyrolactone, dimethyl sulphoxide, tetramethylene sulphone, hexamethylphosphorotriamide and ethylene glycol dimethyl and diethyl ether.

4. Process according to Claims 1 to 3, **characterized in that** the acid catalysts used are sulphuric acid, phosphoric acid, polyphosphoric acids, trifluoroacetic acid, trichloroacetic acid, alkylsulphonic acids, arylsulphonic acids and/or ion exchangers in the H form in an amount of 0.1 to 100% by weight, based on the anhydride defined in Claim 1 used.

5. Process according to Claims 1 to 4, **characterized in that** the stabilizers used are phenol or phenol derivatives in amounts of 0.01 to 5% by weight, based on the anhydride defined in Claim 1 used.

6. Process according to Claims 1 to 5, **characterized in that** the non-aqueous base defined in Claim 1 is used in an amount of 0.5 to 50% by weight, based on the anhydride defined in Claim 1 used.

7. Process according to Claims 1 to 6, **characterized in that** the temperature of the reaction mixture is lowered to 10 to 90°C during or after the addition of the non-aqueous base defined in Claim 1.

8. Process according to Claims 1 to 7, **characterized in that** the N-substituted cyclic imides defined in Claim 1 prepared is obtained from the filtrate present after the separation of the precipitate formed after the addition of the non-aqueous base defined in Claim 1, by evaporation, working-up by distillation or stripping of the solvent and recrystallization of the residue.

9. Process according to Claims 1 to 8, **characterized in that** the acid catalysts used are ion exchangers in the H form or phosphoric acids, which are separated from the reaction mixture and re-used.

10. Process according to Claims 1 to 9, **characterized in that** the filtrate containing the N-substituted cyclic imide defined in Claim 1 is worked up by distillation and the fractions containing solvent and cosolvent are re-used.

## Revendications

1. Procédé pour la préparation d'imides cycliques substitués sur l'atome d'azote, répondant à la formule 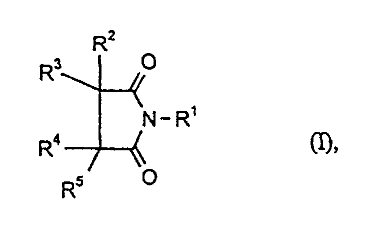 dans laquelle
R¹ représente un radical alkyle en C₁-C₂₀, un radical cycloalkyle en C₃-C₈, un radical alcényle en C₃-C₁₂, un radical aralkyle en C₇-C₁₂ ou un radical aryle en C₆-C₁₀, lesdits radicaux pouvant le cas échéant être substitués, ou représente un radical nitrile, et
R², R³, R⁴ et R⁵ représentent, indépendamment l'un de l'autre, respectivement un atome d'hydrogène ou bien un radical alkyle en C₁-C₁₂ ou un radical alcényle en C₂-C₁₂, lesdits radicaux pouvant le cas échéant être substitués, ou représentent un atome d'halogène, R² et R³ pouvant également représenter ensemble un radical alkylène en C₁-C₆ qui peut le cas échéant être substitué, et R³ et R⁴ pouvant également représenter ensemble une liaison covalente,
avec cette mesure qu'au moins une des trois conditions indiquées ci-dessous est remplie
a) R² et R³ représentent ensemble un radical alkylène en C₁-C₆ qui peut le cas échéant être substitué,
b) R³ et R⁴ représentent ensemble une liaison covalente, et
c) au moins un des radicaux R² à R⁵ représente un radical alcényle en C₂-C₁₂,
d'imides cycliques substitués sur l'atome d'azote répondant à la formule (Ib) 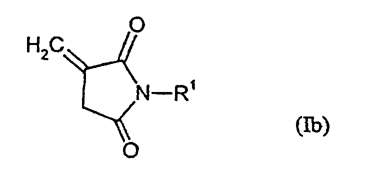 d'imides cycliques substitués sur l'atome d'azote répondant à la formule (Ic) 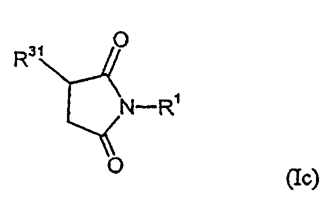 dans laquelle
R³¹ représente un atome de chlore ou un radical alkyle en C₁-C₄,
d'imides cycliques substitués sur l'atome d'azote répondant à la formule (Id) 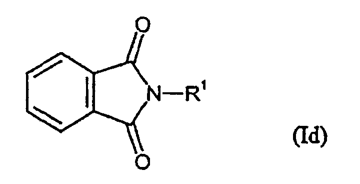 ou de leurs composés soumis à une hydrogénation partielle ou complète,
R¹ dans les formules (Ib), (Ic) et (Id) possédant respectivement la signification indiquée pour la formule (I),
par mise en réaction d'un anhydride d'acide cyclique répondant à la formule 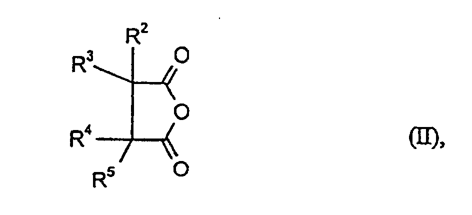 dans laquelle
R² à R⁵ ont la signification indiquée à la formule (I)
de l'anhydride itaconique, de l'anhydride chlorosuccinique, de l'anhydride alkyl(en C₁-C₄)succinique, de l'anhydride phtalique ou d'un anhydride phtalique soumis à une hydrogénation partielle ou complète, avec une amine répondant à la formule
H₂N-R¹ (III)
dans laquelle
R¹ a la signification indiquée à la formule (I),
en présence d'un solvant inerte, non miscible à l'eau et insoluble dans l'eau, avec lequel l'eau qui se forme à la température réactionnelle peut être séparée du mélange réactionnel, et en présence d'un catalyseur acide par une température de 80 à 200 °C, tout en éclusant au dehors l'eau qui s'est formée, **caractérisé en ce qu'**on règle un rapport molaire des anhydrides d'acides cycliques mentionnés ci-dessus à l'amine (III) de 0,5 - 5 : 1 et on effectue le procédé en présence d'un stabilisateur et d'un cosolvant inerte, dipolaire et aprotique, on introduit dans le mélange réactionnel présent après la réaction, le cas échéant un solvant organique inerte, peu polaire ou apolaire, on ajoute une base non aqueuse à propos de laquelle il s'agit de carbonate d'ammonium, d'hydrogénocarbonate d'ammonium, de carbamate d'ammonium, d'urée ou d'ammoniac gazeux, en une quantité de 0,5 à 50 % en poids rapportés à l'anhydride cyclique mis en oeuvre répondant aux définitions indiquées ci-dessus, on sépare le précipité qui se forme et on obtient le filtrat qui contient l'imide cyclique substitué sur l'atome d'azote répondant aux définitions mentionnées ci-dessus.

2. Procédé selon la revendication 1, **caractérisé en ce que** dans les formules (I), (II) et (III), les radicaux alkyle, cycloalkyle, aralkyle, aryle, alcényle et alkylényle portent un ou plusieurs substituants choisis parmi le groupe comprenant un atome de fluor, un atome de chlore, un atome de brome, un atome d'iode, un groupe OH, un groupe NO₂-, un groupe NH₂-, un groupe CN-, un groupe COOH-, un groupe alkyle en C₁-C₄, un groupe alcoxy en C₁-C₄ et/ou un groupe COO(alkyle en C₁-C₆), de préférence **caractérisé en ce que** dans les formules (I) et (III), R¹ représente un groupe alkyle en C₁-C₂₀ non substitué, un groupe cycloalkyle en C₃-C₆ ou un groupe benzyle ou encore un groupe phényle non substitué ou un groupe phényle portant de 1 à 3 substituants choisis parmi le groupe comprenant un groupe alkyle en C₁-C₄, un atome de fluor, un atome de chlore, un groupe nitro, un groupe hydroxyle, un groupe méthoxy et un groupe trifluorométhyle, et
**en ce que** dans les formules (I) et (II)
R² représente un atome d'hydrogène, un groupe alkyle en C₁-C₄, un groupe alcényle en C₃-C₄, un atome de chlore ou un atome de brome et
R³ représente un atome d'hydrogène,
R⁴ représente un atome d'hydrogène ou bien
R³ et R⁴ représentent ensemble une liaison covalente, et
R⁵ représente un atome d'hydrogène, un groupe alkyle en C₁-C₄, un groupe alcényle en C₃-C₄, un atome de chlore ou un atome de brome,
procédé dans lequel, soit au moins un des radicaux R² et R⁵ représente un groupe alcényle en C₃-C₄, soit R³ et R⁴ représentent ensemble une liaison covalente, soit R² et R³ représentent ensemble un groupe alkylène en C₁-C₄, R⁴ représente un atome d'hydrogène et R⁵ représente un atome d'hydrogène, un groupe alkyle en C₁-C₄, un groupe alcényle en C₃-C₄, un atome de chlore ou un atome de brome.

3. Procédé selon les revendications 1 et 2, **caractérisé en ce qu'**on met en oeuvre, à titre de solvant, le benzène, le toluène, des xylènes, le cumène, le mésitylène, l'éthylbenzène, le butylbenzène, le i-propylméthylbenzène, le t-butylbenzène, la Tétraline, la Décaline, le chlorobenzène, les dichlorobenzènes ou l'anisol, ainsi que n'importe quels mélanges de ces solvants les uns avec les autres, avec addition d'un cosolvant à concurrence de 0,1 à 20 % en poids, qui est choisi parmi le groupe comprenant la N-méthylpyrrolidone, le dioxanne, le formamide, le N-méthylformamide, le diméthylformamide, le diméthylacétamide, la tétraméthylurée, la N-méthylcaprolactone, la butyrolactone, le diméthylsulfoxyde, la tétraméthylènesulfone, le triamide de l'acide hexaméthylphosphorique et l'éther diméthylique et diéthylique d'éthylèneglycol.

4. Procédé selon les revendications 1 à 3, **caractérisé en ce qu'**on met en oeuvre, à titre de catalyseurs acides, l'acide sulfurique, l'acide phosphorique, des acides polyphosphoriques, l'acide trifluoroacétique, des acides alkylsulfoniques, des acides arylsulfoniques et/ou des échangeurs d'ions sous la forme H en une quantité de 0,1 à 100 % en poids rapportés à l'anhydride mis en oeuvre répondant aux définitions mentionnées à la revendication 1.

5. Procédé selon les revendications 1 à 4, **caractérisé en ce qu'**on met en oeuvre, à titre de stabilisateurs, du phénol ou des dérivés du phénol dans des quantités de 0,01 à 5 % en poids rapportés à l'anhydride mis en oeuvre répondant aux définitions mentionnées à la revendication 1.

6. Procédé selon les revendications 1 à 5, **caractérisé en ce qu'**on met en oeuvre la base non aqueuse définie à la revendication 1 en une quantité de 0,5 à 50 % en poids rapportés à l'anhydride mis en oeuvre répondant aux définitions mentionnées à la revendication 1.

7. Procédé selon les revendications 1 à 6, **caractérisé en ce que**, pendant ou après l'addition de la base non aqueuse définie à la revendication 1, on abaisse la température du mélange réactionnel de 10 à 90 °C.

8. Procédé selon les revendications 1 à 7, **caractérisé en ce qu'**on récupère les imides cycliques préparés substitués sur l'atome d'azote répondant aux définitions mentionnées à la revendication 1, à partir du filtrat que l'on obtient après la séparation du précipité formée après l'addition de la base non aqueuse définie à la revendication 1, par évaporation, par traitement par distillation ou par extraction du solvant et recristallisation du résidu.

9. Procédé selon les revendications 1 à 8, **caractérisé en ce qu'**on utilise, à titre de catalyseurs acides, des échangeurs d'ions dans la forme H ou des acides phosphoriques, on les sépare du mélange réactionnel et on les met à nouveau en oeuvre.

10. Procédé selon les revendications 1 à 9, **caractérisé en ce qu'**on traite par distillation le filtrat qui contient l'imide cyclique substitué sur l'atome d'azote répondant aux définitions mentionnées à la revendication 1 et on recycle les fractions contenant le solvant et le cosolvant.
